# EUROPEAN PATENT APPLICATION

(11) **EP 2 157 173 A1**
(43) Date of publication of application: **24.02.2010**
(21) Application number: 09005619.3
(22) Date of filing: 18.12.2002
(51) Int. Cl.: C12N 7/00

(54) **An avian embryo particulate biomass for the production of virus antigens**

(30) Priority: 21.12.2001 US 343339 P
(62) Divisional of application: 02795935.2
(71) Applicant: Wyeth, Madison, NJ 07940 (US)
(72) Inventor: Wederquist, David Lou, Lee's Summit MO 64081 (US)
(74) Representative: Denholm, Anna Marie

(57) **Abstract**

The present invention provides a biomass which comprises avian embryonic particles having a particle size of about 0.5 mm to 10.0 mm and the use thereof in a method for the production of virus antigens. Also provided is a method for the preparation of a vaccine useful for the amelioration and prevention of viral disease.

## Description

### BACKGROUND OF THE INVENTION

Conventional methods for producing a virus antigen include *in ovo* production i.e., production within an infected embryonated egg; or cell culture production i.e., primary cells or a cell line which have been infected. A typical *in ovo* method for producing antigen involves many steps which are difficult to automate and are labor intensive; time consuming and subject to contamination. In general, cell culture production entails the use of individual cells and cell aggregates which are as small as possible and which require the tissue to be disintegrated or disassociated to the utmost extent mechanically or enzymatically. This treatment may lead to the decay of many cells which may result in a high degree of contamination of cell proteins which are difficult to separate from the desired product. Methods for producing tick-born encephalitis virus antigen and influenza virus vaccine are described in US 5,391,491 and US 5,698,433, respectively. These methods use avian embryo cell aggregates having a diameter of >100 µm to <1,000 µm and require an enzymatic step.

Therefore, it is an object of this invention to provide a biomass useful for the production of virus antigens which eliminates the disadvantages of *in ovo* antigen production and antigen production using individual cells, cell lines or cell aggregates of micron dimensions.

It is another object of this invention to provide a method for the production of virus antigens which leads to high production output of virus antigen and which may be used on a commercial scale.

It is an advantage of this invention that the biomass is easy to handle and the antigen production method offers an economy of steps and significantly reduced opportunity for contamination.

It is a feature of this invention that vaccines effective against viral infection and disease may be more readily and economically produced.

Further objects and features of the invention will become more apparent from the detailed description set forth hereinbelow.

### SUMMARY OF THE INVENTION

The present invention provides a biomass for producing virus antigen which comprises avian embryo particles having a particle size of about 0.5 mm to 10.0 mm wherein said particles are infected with a virus.

The present invention also provides a method for the production of a virus antigen which comprises:
a) infecting avian embryo particles having a particle size of about 0.5 mm to 10.0 mm with a virus in a culture medium to form a biomass;
b) oxygenating said biomass at an elevated temperature to form an oxygenated mixture; and
c) filtering said mixture to give a filtrate containing the desired virus antigen product.

The present invention further provides a method for the preparation of a vaccine which comprises:
a) infecting avian embryo particles having a particle size of about 0.5 mm to 10.0 mm with a virus in a culture medium to form a biomass;
b) oxygenating said biomass at an elevated temperature to form an oxygenated mixture;
c) filtering said mixture to give a filtrate containing the desired virus antigen product;
d) mixing said filtrate with a pharmacologically acceptable liquid carrier; and
e) optionally adding an immunogenically stimulating adjuvant.

### DETAILED DESCRIPTION OF THE INVENTION

Conventional methods for producing a virus antigen include production in an infected embryonated egg such as a hen's egg, production in a primary cell culture which has been infected or on an infected cell line or production using avian embryo cell aggregates of micron dimensions. All of these methods involve multiple steps and/or enzymatic cleavage and separation techniques such as centrifugation, sedimentation, or the like.

Surprisingly, it has now been found that a biomass comprising avian, preferably chicken, embryo particles having a particle size of about 0.5 mm to 10.0 mm, preferably about 1.0 mm to 3.0 mm, wherein said particles are infected with a virus may be used to effectively and efficiently produce a virus antigen. The avian embryo particles of the biomass according to the invention may be obtained by conventional mechanical size reduction methods such as high shear blending, rapid multi-baffled stirring, homogenizing or the like, preferably homogenizing. For example, chicken embryos which.have been harvested from 9-12, preferably 11, day old incubated hen eggs may be washed with a sterile buffer solution, diluted with a culture medium such as tryptose phosphate broth to a concentration of about 50 to 250, preferably about 80-120, more preferably about 100, embryos per liter and mechanically reduced in size to give a suspension of avian embryo particles having a particle size of about 0.5 mm to 10.0 mm, preferably 1.0 mm to 3.0 mm. This suspension may then be infected with a virus or virus master seed to give a biomass in accordance with the invention. Suitable viruses include any virus or other antigen capable of reproducing in avian embryonic cells, such as Reovirus, Infectious Bursal Disease Virus (IBDV), Marek's Disease Virus (MDV), Newcastle Disease Virus (NDV), Infectious Bronchitis Virus (IBV), Poxvirus, Chicken Anemia Virus (CAV), Egg Drop Syndrome (EDS), Turkey Rhinotracheitis (TRT) Virus, Pneumovirus, Infectious Laryngotracheitis (ILT) Virus, Encephalomyelitis Virus, Influenza Virus, Rabies Virus, Distemper Virus, Hemorrhagic Enteritis Virus, Hepatitis Virus, Chlamydia Psittaci, Haemophilus Paragallinarum, or the like, preferably avian viruses, more preferably Infectious Bursal Disease Virus.

Accordingly, the present invention provides a method for the production of virus antigen which comprises infecting avian embryo particles having a particle size of about 0.5 mm to 10.0 mm, preferably about 1.0 mm to 3.0 mm, with a virus, preferably an avian virus, more preferably infectious bursal disease virus, in a culture medium to form a biomass; oxygenating said biomass at an elevated temperature to form an oxygenated mixture; and filtering said mixture to give a filtrate containing the desired virus antigen product.

Culture media suitable for use in the method of invention include tryptose phosphate broth, EMEM, DMEM, (manufactured by Anhui Chemicals) or any conventional media suitable for biological cultures, preferably tryptose phosphate broth.

Elevated temperatures suitable for use in the method of invention are temperatures of about 90°F to 110°F, preferably about 95°F to 105°F, more preferably about 98°F to 102°F.

Oxygenated mixtures obtained in the method of the invention may contain about 40% to 60%, preferably 45% to 55%, more preferably 50%, dissolved oxygen.

In actual practice a suspension of avian embryo particles having a particle size of about 0.5 mm to 10.0 mm, preferably about 1.0 mm to 3.0 mm, in a culture medium such as tryptose phosphate broth having a concentration of about 50 to 250, preferably about 80-120, more preferably about 100 embryos per liter of culture medium is infected with a virus, preferably an avian virus, more preferably infectious bursal disease virus, to give a biomass; said biomass is oxygenated at an elevated temperature of about 90°F to 110°F, preferably about 95°F to 105°F, more preferably about 98°F to 102°F, to give an oxygenated mixture having about 40% to 60%, preferably about 45% to 55%, more preferably about 50% dissolved oxygen; this oxygenated mixture is filtered through a screen of about 50 micron to 100 micron, preferably about 70 micron to 80 micron, more preferably about 75 micron, to obtain a filtrate containing the desired antigen product. The antigen stock thus-obtained may be treated with conventional excipients such as stabilizers, antioxidants, antifoam agents, or the like and stored via freezing or lyophilazation for use in future vaccine preparation or may be used as is in a vaccine preparation.

Accordingly, the present invention also provides a method for the preparation of a vaccine which comprises mixing the antigen stock produced by the method described hereinabove with a pharmacologically acceptable carrier and optionally adding an immunogenically stimulating adjuvant.

Pharmacologically acceptable carriers suitable for use in the vaccine preparation method of the invention include any conventional liquid carrier suitable for veterinary pharmaceutical preparations, preferably a balanced salt solution suitable for use in tissue culture media.

Immunogenically stimulating adjuvants suitable for use in the vaccine preparation method of the invention include any compound which is capable of potentiating or stimulating an immune response in an animal when administered in combination with an antigen such as surfactants, i.e. as hexadecylamine, octadecylamine, lysolecithin, dimethyl dioctadecyl ammonium bromide, N,N-dioctadecyl-N'-N-bis(2-hydroxyethyl-propane diamine), methoxyhexadecylglycerol, Pluronic^{®} polyols, saponin, Quil^{®} A, or the like; polyanions such as pyran, dextran sulfate, polynucleotide complex of polyinosinicpolycytidylic acid, polyacrylic acid, carbopol, aluminum hydroxide, aluminum phosphate, or the like; peptides such as muramyl dipeptide, dimethyl glycine, tuftsin or the like; oil emulsions; immunomodulators such as interleukin-1, interleukin-2, interleukin-12, GM-CSF or the like; or a combination thereof.

In actual practice, virus antigen stock preferably avian virus antigen, more preferably infectious bursal disease virus antigen, obtained according to the antigen production method of the invention as described hereinabove is mixed with a pharmacologically acceptable carrier such as phosphate buffered saline solution and optionally an immunogenically stimulating adjuvant to give a vaccine product having an antigen concentration of about 1.2 log above the minimum protective dose.

The vaccine thus prepared may be further treated with conventional excipients commonly used in veterinary vaccines such as stabilizers, antioxidants, antifoam agents or the like.

In one embodiment of the invention the virus antigen stock may be inactivated prior to the vaccine preparation. The virus antigen stock prepared according to the antigen production method of the invention may be inactivated by conventional inactivating means, for example chemical inactivation using chemical inactivating agents such as binary ethyleneimine, beta-propiolactone, formalin, merthiolate, glutaraldehyde, sodium dodecyl sulfate, or the like, or a mixture thereof, preferably formalin. Said antigen may also be inactivated by heat or psoralen in the presence of ultraviolet light.

For a more clear understanding of the invention, the following examples are set forth below. These examples are merely illustrative and are not understood to limit the scope or underlying principles of the invention in any way. Indeed, various modifications of the invention, in addition to those shown and described herein, will become apparent to those skilled in the art from the following examples and the foregoing description. Such modifications are also intended to fall with the scope of the appended claims.

Unless otherwise noted, all parts are parts by weight.

### EXAMPLE 1

### Preparation of Infectious Bursal Disease Virus (IBDV) Antigen

Embryos from hen eggs which have been incubated at 99°F for 11 days are harvested, washed three times with a solution of gentamicin in phosphate buffer solution (30 mg/mL) at room temperature. The washed embryos are diluted to approximately 100 embryos per liter in tryptose phosphate broth. The diluted embryos are homogenized to a particle size of 1.0 to 3.0 µm using a W250V (Gifford-Wood) model homogenizer, manufactured by Chemineer (Greerco) with a preset gap setting of 3.0 mm. The resultant suspension is mechanically mixed with 0.2 mL per embryo of X+4 *Lukert* strain working seed (USDA-APHIS approved IBDV)(5.5 TCID₅₀/mL) for 20-30 minutes. The resultant mixture is oxygenated at pH 7.1, 3.0 psi, 100.4°F and a concentration of 20 embryos/Liter to a final dissolved oxygen (DO) content of 50% DO. After 48h, the oxygenated mixture is filtered through a 75 micron screen, mixed 1:1 with Stabilizer H¹ for 1 h at room temperature and stored at ≤ 40°F.

Using the above procedure, IBDV antigen stock having a potency of 9.2 TCID₅₀/mL is obtained. TCID designates tissue culture infectious dose.
¹Stabilizer H formulation: The below-listed ingredients are admixed in the order listed. Each ingredient is dissolved completely before the next is added. Heat to a maximum of 60°C is applied as needed.

| **Ingredient** | **wt/vol** |
|---|---|
| Purified water | 0.5 kg/L |
| Pharmatone, manufactured by American Labs | 5.0 g/L |
| Peptone Bacto, manufactured by Becton Dickinson | 45.0 g/L |
| Sucrose | 50.0 g/L |
| N-Z-Amine Type Yt, manufactured by Quest International | 25.0 g/L |
| Monosodium glutamate | 5.0 g/L |
| Purified water | Q. S. |

### EXAMPLE 2

### Preparation of Virus Antigens

Using essentially the same procedure described in Example 2 and employing the appropriate virus, the antigen stocks shown in Table I are obtained.

**Table I**

| **Antigen** | **Potency** |
|---|---|
| Reovirus | 8.26 TCID₅₀/mL |
| Infectious Bursal Disease Virus | 9.20 TCID₅₀/mL |
| Newcastle Disease Virus | 8.60 EID¹₅₀/mL |
| ¹EID = Embryo Infectious Dose | |

### EXAMPLE 3

### Preparation of Infectious Bursal Disease Virus Vaccine

Infectious bursal disease antigen stock, which has been prepared according to the procedure described in Example 1 and frozen, is thawed at room temperature and diluted with a 1:1 mixture of stabilizer H and D-mem¹ to a final antigen concentration of 1.2 log above the minimum protective dose. The diluted stock is mechanically stirred for at least 15 minutes and placed in single-, or multi-, dose vials.
¹D-mem is minimum essential medium manufactured by JRH Bioscience.

## Claims

1. A biomass for producing virus antigen which comprises avian embryo particles having a particle size of about 0.5 mm to 10.0 mm wherein said particles are infected with a virus.

2. The biomass according to claim 1 wherein the particle size is about 1.0 mm to 3.0 mm.

3. The biomass according to claim 1 wherein said avian embryo is a chicken embryo.

4. The biomass according to claim 1 wherein said virus is selected from the group consisting of Reovirus; Infectious Bursal Disease Virus; Marek's Disease Virus; Newcastle Disease Virus; Infectious Bronchitis Virus; Poxvirus; Chicken Anemia Virus; Egg Drop Syndrome; Turkey Rhinotracheitis Virus; Pneumovirus; Infectious Laryngotracheitis Virus; Encephalomyelitis Virus; Influenza Virus; Rabies Virus; Distemper Virus; Hemorrhagic Enteritis Virus; Hepatitis Virus; Haemophilus Paragallinarum; and Chlamydia Psittaci.

5. The biomass according to claim 1 wherein said virus is an avian virus.

6. The biomass according to claim 5 wherein said virus is Infectious Bursal Disease Virus.

7. A method for the production of a virus antigen which comprises:
a) infecting avian embryo particles having a particle size of about 0.5 mm to 10.0 mm with a virus in a culture medium to form a biomass;
b) oxygenating said biomass at an elevated temperature to form an oxygenated mixture; and
c) filtering said mixture to give a filtrate containing the desired virus antigen product.

8. The method according to claim 7 wherein said embryo particle size is about 1.0 mm to 3.0 mm.

9. The method according to claim 7 wherein said culture medium is tryptose phosphate broth.

10. The method according to claim 7 wherein said oxygenated mixture contains about 40% to 60% dissolved oxygen.

11. The method according to claim 7 wherein the elevated temperature is about 95°F to 105°F.

12. The method according to claim 7 wherein the virus is selected from the group consisting of Reovirus; Infectious Bursal Disease Virus; Marek's Disease Virus; Newcastle Disease Virus; Infectious Bronchitis Virus; Poxvirus; Chicken Anemia Virus; Egg Drop Syndrome; Turkey Rhinotracheitis Virus; Infectious Laryngotracheitis Virus; Encephalomyelitis Virus; Influenza Virus; Rabies Virus; Distemper Virus; Enteritis Virus; Hepatitis Virus and Chlamydia Virus.

13. The method according to claim 7 wherein the virus is an avian virus.

14. The method according to claim 13 wherein the virus is infectious bursal disease virus.

15. A method for the preparation of a virus vaccine which comprises:
a) infecting avian embryo particles having a particle size of about 0.5 mm to 10.0 mm with a virus in a culture medium to form a biomass;
b) oxygenating said biomass at an elevated temperature to form an oxygenated mixture;
c) filtering said mixture to give a filtrate containing the desired virus antigen product;
d) mixing said filtrate with a pharmacologically acceptable liquid carrier; and
e) optionally adding an immunogenically stimulating adjuvant.

16. The method according to claim 15 wherein said virus is selected from the group consisting of Reovirus; Infectious Bursal Disease Virus; Marek's Disease Virus; Newcastle Disease Virus; Infectious Bronchitis Virus; Poxvirus; Chicken Anemia Virus; Egg Drop Syndrome; Turkey Rhinotracheitis Virus; Infectious Laryngotracheitis Virus; Encephalomyelitis Virus; Influenza Virus; Rabies Virus; Distemper Virus; Enteritis Virus; Hepatitis Virus and Chlamydia Virus.

17. The method according to claim 15 wherein said virus is an avian virus.

18. The method according to claim 17 wherein said virus is Infectious Bursal Disease Virus.

19. The method according to claim 18 wherein said avian embryo particles have a particle size of about 1.0 mm to 3.0 mm.

20. The method according to claim 19 wherein said particles are chicken embryo particles
